# EUROPEAN PATENT APPLICATION

(11) **EP 3 124 500 A1**
(43) Date of publication of application: **01.02.2017**
(21) Application number: 15770271.3
(22) Date of filing: 27.03.2015
(51) Int. Cl.: C07K 16/30, G01N 33/53, C07K 7/08

(54) **ANTI-GRPR ANTIBODY, METHOD FOR PRODUCING SAME, DETECTION METHOD, USE OF THE ANTIBODY, KIT AND GENE CONSTRUCT**

(30) Priority: 27.03.2014 BR 14007315
(71) Applicant: Ziel Biosciências Pesquisa, Desenvolvimento e Diagnostico Ltda, 91501-970 Porto Alegre (BR)
(72) Inventor: CORNELIO, Daniela Bauman, 90560-002 Porto Alegre (BR); DE FARIAS, Caroline Brunetto, 90050-170 Porto Alegre (BR)
(74) Representative: Carvajal y Urquijo, Isabel
(86) International application number: PCT/BR2015/050034
(87) International publication number: WO 2015/143525

(57) **Abstract**

The present invention relates to an anti-GRPR antibody obtained from a process comprising the exposure step of at least one animal to a peptide with at least 60% identity to the sequence SEQ ID NO: 1. The present invention also describes a process for obtaining anti-GRPR antibody, an in vitro method for detection of dysplastic and / or neoplastic cervical lesions, the use of anti-GRPR antibody, a kit for the qualitative detection and / or quantitative detection of dysplastic and / or neoplastic cervical lesions, and a genetic construct for expression or production of antigenic peptide. The present invention lies in the fields of Medicine, Immunology and Molecular Biology.

## Description

### Field of Invention

The present invention lies in the fields of Medicine, Immunology and Molecular Biology. More specifically, the present invention relates to an anti-GRPR antibody obtained from a process comprising the exposure step of at least one animal to a peptide with at least 60% identity to the sequence SEQ ID NO: 01. This invention also describes a process for obtaining an anti-GRPR antibody, an in vitro detection method of dysplastic and / or uterine neoplastic lesions, the use of anti-GRPR antibody, a kit for the qualitative detection and / or quantitative detection of dysplastic and / or neoplastic uterine lesions, and a genetic construct for expression or production of antigenic peptide.

### Background of the Invention

Cervical cancer is the second most common cancer in women worldwide, with about 500,000 new cases each year. The incidences are higher in developing countries where cervical cancer is the leading cause of mortality in female cancer. In
According to the American Cancer Society, "Mortality from cervical cancer has declined more than 70 percent in recent decades due to the introduction of the Pap test." Cervical cancer, which has been the main cause of cancer deaths in women, is now the thirteenth in the most lethal tumors list in women in the United States.

The Pap smear is performed by morphological analysis of cervical cells. The intent of screening is not just to detect cervical cancer, but also to detect high-grade lesions, preventing progression to invasive cancer. As the screening by cervical cytology has become more prevalent, pre-invasive lesions of the uterine cervix are now detected more often than invasive tumors. In women detected and treated with pre-invasive lesions, the survival rate at 5 years is almost 100 percent. Even those who have already progressed to invasive tumors, when they are detected at early stages, the survival rates in 5 years are still approximately 92 percent. In this way, cervical cancer is one of the few tumors that can be virtually cured. Therefore, the search for more accurate tests (with less false negatives) is a priority to save lives.

Despite the significant reduction in the incidence and mortality from cancer of the cervix in recent decades, many women are still not properly screened, because of the low coverage of Pap tests or due to the limitations of this method. The effectiveness of the Pap test is hampered by the high rate of false positives and false negatives, and a single test only 50% sensitive in detecting high-grade lesions or invasive carcinoma.

About 50 million Pap tests are performed each year in the United States. Approximately 7 percent, or 3.5 million, exhibit abnormalities that require follow-up investigations. Among these 7 percent, 2 million are diagnosed as ASCUS or ASC-H, 1.25 million as low-grade lesions, 300,000 as high-grade lesions and 13,000 as invasive cancers. Unfortunately, false negative are hidden among millions of women who are declared as normal or negative for cancer.

Almost half of cervical cancer detected in United States occur in women who have never been screened, and 10 percent in those that were not screened in the past 5 years. There are, therefore, almost 40 percent of cases occurring in women who were falsely diagnosed as negative for cancer. The false negative results occur even in optimized screening programs and cannot be eliminated entirely.

### The National Breast and Cervical Cancer Early Detection Program

(NBCCEDP) reported that a negative Pap test woman has 5 per percent chance to progress to injury in a year; this risk doubles for the following year. The College of American Pathologists stated that "The Pap smear is a screening test that involves subjective interpretations of thousands of cells that are present in a cytological gynecologic specimen by a cytology technician or pathologist." Studies indicate an irreducible rate of false-negatives of about 5 percent. Although frequent screening can reduce the false negative rates, you cannot get results free of errors with this technique today.

In 1996, the Consensus Conference on Cervical Cancer of the National Institute of Health (NIH) of the United States revealed that one in five women with cervical cancer had a negative Pap test within the 5 years prior to the occurrence of cancer. This 20% rate of false-negative was considered unacceptable by the NIH, and major improvements in the test were required. Great efforts have been invested in this direction. US government policies have been launched to establish improvements in Pap test and empower providers and facilitators, professional societies have developed guidelines and accreditations to protect these policies and the medical industry started to develop tools to improve the quality of Pap smear.

The American Cancer Society, a professional association that invests in promoting Pap smear since 1950 until today, recently revised its guidelines for the early detection of cancer. This document recognized the value of new technologies for the screening of cervical cancer, suggesting that new, more accurate methods are needed.

In recent years, improvements have been attempted in the art of Pap test such as liquid based cytology. However this did not improve the sensitivity and specificity for the detection of cervical intraepithelial neoplasia compared with conventional cytology. There were also evaluated many molecular methods for detection of cervical cancer, including HPV testing.

HPV is a sexually transmitted virus that infects 50 percent of the population at some point in the course of life, and their prolonged infection is associated with cervical cancer. There are more than 90 types of HPV, but only some of them are responsible for the development of cancer. Vaccines against HPV infection have been developed, but their real effect on reducing cancer cannot yet be estimated. Although there is a potential benefit for the prevention of cervical cancer, vaccines will not modify the screening scenario, at least in the next few decades. HPV tests have been approved by the Food and Drug Administration (FDA) as complementary to Pap smears, especially in doubtful cases, as ASCUS results, and more recently are being used as primary screening test.

It is known that up to 10% of Pap tests are classified as ASCUS (atypical squamous cells of undetermined significance), that is, you cannot make a clear categorization of no neoplastic, moderate or severe lesion, or tumor. However, experience shows that up to 10% of this ASCUS population has high-grade lesions, which may go unnoticed. According to the American Society for Colposcopy and Cervical Pathology, facing a cytological abnormal type ASCUS, consensus guidelines recommend repeating the Pap test, perform colposcopy or HPV testing. Since 31-60% of patients with questionable Pap smears have HPV, preferably they should perform colposcopy.

Other molecular markers that have been studied are Ki-67 and p16INK4a (p16) (for example, in US patent 7,455,973). The inhibitor of cyclin-dependent protein kinase, p16, is considered a marker for the oncogenic activity of HPV in cervical cells and their overexpression is well established in CIN and invasive cancer. The Ki-67 is a marker of cell proliferation. Both these tests are already used in cases of dubious Pap tests, but are not recommended for routine use in screening for cervical cancer.

Thus, considering the context of what has been stated above, searching alternative technologies for screening and diagnosis of cervical cancer is essential. In this aspect, the present invention comes directly to meet these efforts.

The gastrin releasing peptide (GRP) is a neuroendocrine peptide which was shown to have stimulatory effect on the growth in many types of cancer. The preferred GRP receptor (GRPR) receptor belongs to the family of G-protein and activates multiple signal transduction pathways, resulting in cell proliferation and growth. GRPR is overexpressed in a wide variety of human tumors, including prostate, breast, ovary, lung, head and neck, stomach, colon, esophagus and kidney cancer.
Our group recently described the aberrant expression of GRP receptors in cervical dysplasia and invasive carcinoma. Based on the presence of this receptor in 99% of analyzed samples, but not in malignant cervical tissue, it was suggested that GRPR may play a role in neoplastic cervical processes.

The search in the patent literature pointed out some partially relevant documents, which will be described below.

WO 2003/097666 describes the use of gastrin releasing peptide (GRPR) and its receptor, commonly found in stomach cells, in the diagnosis of colon, prostate, stomach and pancreatic cancer. The document also cites a diagnostic kit containing anti GRPR and primers for amplification and hybridization GRPR in patients potentially with cancer. The present invention differs from this document, among other technical reasons, because they involve the use of anti-GRPR antibodies in a method for the detection of GRPR and diagnostic kit in cervical lesions, facts not mentioned or suggested in the state of the art. By this document one may also assume that the expected by a skilled in the art would be to identify GRPR in cancers associated with stomach lesions, and no cervical lesions, as described herein.

US 7,455,973 discloses a cervical disease detection method by immunocytochemistry technique. The present invention differs from that document, among other technical reasons for not making any reference or suggestion to the detection of GRPR.

The KR 20060064049, WO 2009/143101, PI 0412824 and PI 0317987 disclose pharmaceutical compositions comprising GRPR and modified gastrin releasing compounds. The present invention differs from those documents, among other technical reasons, to present a new use of anti-GRPR antibodies and a kit and method for the detection / diagnosis of cervical injuries not described in the documents mentioned above. The four (4) documents referred to above relate to radiopharmaceutical formulations that target GRPR and do not relate to the immunocytochemical or immunohistochemical techniques.

The document CN 101368966 A refers to a gastrin releasing peptide, chemiluminescent immunoassay kit said kit and method of preparation. The kit is used for early diagnosis of small cell lung cancer. The present invention differs from that document, among other technical reasons, for providing an immunoassay diagnostic kit of neoplastic and / or dysplastic lesions including cervical intraepithelial neoplasia as well as cervical cancer.

The article "Influence of GRPR and BDNF / TrkB signaling on the viability of breast cancer and gynecologic cells "(CORNELIO et al., 2013), in which the present inventor is one of the authors, shows that GRPR activation reduces the viability of specific cell lines of breast, ovarian and cervical cancer, while the GRPR blocking increases the viability of such cell lines. However, nothing is disclosed or suggested in that article about the antibody of the invention, nor provide information leading a technician in the matter to reach a kit or immunoassay procedure for diagnosis of neoplastic lesions and / or dysplastic lesions including cervical cancer.

Thus, based on the patent and non-patent literature, it is clearly noticed the need to search for new alternatives to those already existing for the detection of neoplastic and / or dysplastic lesions, in particular for the detection of cervical intraepithelial neoplastic lesions and cervical cancer using anti-GRPR antibodies disclosed in the present patent application.

It is noteworthy that in the current state of the art, no molecular marker is used for screening. There are only markers that are used to aid the diagnosis by the pathologist, improving the cytopathological and pathological diagnosis, different from the invention now proposed. In the present invention GRPR can be used not only as an aid in the laboratory diagnosis of pathology but also be implemented for screening through a rapid test (e.g. in a kit), providing immediate results that the patient may have a lesion.

Through the search of the literature, no documents were found suggesting or anticipating the teachings of the present invention. In the eyes of the inventors, this invention has novelty and inventive activity in relation to the state of the art.

### Summary of the Invention

The present invention has the inventive concept, common to its various objects, the use anti-GRPR antibody to prepare kits, or in processes comprising the binding, detection and / or quantification of GRPR receptors in biological tissues or cells, for the diagnosis of dysplastic and / or cervical neoplastic lesions, including cervical intraepithelial neoplasia and cervical cancer.

The present invention provides numerous advantages over the state of the art as, for example, provides a useful rapid test for screening. It can also assist the diagnosis by pathologists; in case of biological samples positive for the expression of GRPR, the pathologist should look more closely at the changes in cytopathology and / or pathologic exams.

It is an object of the present invention an anti-GRPR antibody obtained from a process comprising the steps of:
a) exposing at least one animal to a peptide with at least 60% identity to the sequence SEQ ID NO: 1; and
b) obtaining anti-GRPR antibodies after the animal immune response to said peptide of step a).

It is another object of the present invention an anti-GRPR antibody obtained from a process comprising the steps of:
a) exposing at least one animal to a peptide with at least 60% identity to the sequence SEQ ID NO: 1; and
b) obtaining anti-GRPR antibodies after the animal immune response to said peptide of step a).

It is another object of the present invention a process / method of in vitro detection of dysplastic and / or neoplastic cervical lesions comprising the steps of:
a) contacting at least one anti-GRPR antibody as disclosed by the present invention with at least one sample of biological material of uterine origin; and
b) detecting binding of said antibody to one or more GRPR receptors present in the said biological material.

It is another object of the present invention the use of anti-GRPR antibody as disclosed by this invention for the preparation of kits for the detection and / or quantification of GRPR receptors in biological tissues or cells, being the kit for diagnosis of dysplastic and / or neoplastic lesions.

It is another object of the present invention a kit for the qualitative and / or quantitative detection of dysplastic and / or neoplastic cervical lesions comprising anti-GRPR antibody as disclosed by the present invention and at least one additional reagent for detecting the binding of the said antibody with GRPR receptor(s) present in tissues, cells, or biological fluids.

It is another object of the present invention, a genetic construct for expression or production of antigenic peptide, said gene construct comprising:
a) a nucleotide sequence encoding a polypeptide having identity equal to or higher than 60% to SEQ ID NO: 1;
b) a promoter for the said nucleotide sequence; and
c) a nucleotide sequence selected from: a nucleotide transcription terminator sequence; a marker selection; a sequence secretion signal; a sequence that facilitates the export or purification; a nucleotide sequence encoding another polypeptide antigen sequence; combinations thereof or a plasmid comprising sequences, being heterologous any of the nucleotide sequences defined above.

It is another object of the present invention a process / method of obtaining antibodies which comprises the steps of:
a) inoculating the gene construction as disclosed in this patent application in at least one animal; and
b) obtaining antibodies in said animal plasma after the immune response of the animal.

These and other objects of the invention will be immediately appreciated by those versed in the art and by companies with interests in the sector, and will be described in sufficient detail for their reproduction in the description below.

### Brief Description of the Figures

Figure 1 shows the analysis by high pressure liquid chromatography (HPLC) of an embodiment of the antigenic peptide of the invention (SEQ ID NO: 1) synthesized for production of anti-GRPR antibody.
Figure 2 shows the analysis by mass spectrometry of an embodiment of the antigenic peptide invention (SEQ ID NO: 1) synthesized for production of anti-GRPR antibody.
Figure 3 shows the results of dot blot of serum of the rabbit immunized with the peptide referred to in Figures 1 and 2, conjugated to KLH and tested against peptide conjugated to albumin. A) serum diluted 1: 200; B) serum diluted 1: 2000.
Figure 4 demonstrates the comparison of the primary antibodies (obtained from the peptide of SEQ ID NO: 1 x control antibody (restricted use in
   research) relative to GRPR expression by immunohistochemistry in non-neoplastic cervical samples. A) Anti-GRPR antibody of this invention (1: 200) and B) Anti-GRPR control antibody (1:50). Both in increase 20x.
Figures 5 to 7 comparatively evaluate the primary antibodies of the invention x control (restricted use in research) in relation to GRPR expression by immunohistochemistry in cervical samples with cervical intraepithelial lesion II and III (CIN II and III). A) Anti-GRPR antibody of this invention (1: 200) and B) Anti-GRPR control antibody (1:50). Figures 5 and 6: Increased 20x, Figure 7: Increased 40X.
Figure 8 illustrates a schematic drawing of a strip for immunochromatographic test. The top side refers to the lateral view the tape, being indicated: in 1 the portion of the sample; in 2 the absorbing portion; in 3 a nitrocellulose membrane; in 4 the test line; in 5 the control line. The bottom view shows the strip top view, the arrows indicating the flow direction. On said nitrocellulose membrane are inserted: the initial portion that receives the sample (sample pad), the final portion which absorbs the sample (absorbent pad), the test line and the control line.
Figure 9 shows a schematic drawing of a strip for immunochromatographic lateral flow test using FUSION5 ™ membrane. The red arrow indicates the location of application of the conjugated of colloidal gold with the antibody anti-GRPR (T, test line) and the dark arrow indicates the site of application of IgG secondary goat anti-rabbit antibody conjugated to colloidal gold (C, control line).
Figure 10 shows an example of immunochromatographic lateral flow test using the anti-GRPR antibody of the invention. In a: control strip, being added only running buffer. B and c: test strips, being added solution containing sample positive for GRPR after 1 and 5 minutes, respectively.
Figures 11 a and 11 b show a schematic representation of an embodiment of the gene construct of the invention. In Figure 11A we see the complete sequence of the peptide analyzed by the antigenic index. Peaks indicate the most immunogenic regions. Figure 11b is can be visualized the most immunogenic region comprised in the sequence ESTNQTFISCAPYPHSN (Glu-Ser-Gln-Asn-Thr-Phe-Thr-Ser-Ile-Cys-Ala-Pro-Tyr-Pro-Ser-His-Asn), which was chosen for gene construct of the invention.
Figure 12 shows a Western blot assay demonstrating the presence of anti-GRPR antibody, which can be seen in the bands as indicated by the arrows.
The figures 13a and 13b show the evaluation by immunohistochemistry with the anti-GRPR purified antibody compared to hematoxylin-eosin.
Figure 14 shows the immunohistochemical evaluation comparing a) Hematoxylin-eosin b) purified anti-GRPR antibody of the present invention and c) the commercial antibody anti-GRPR. The arrow indicates intense nuclear staining in high-grade neoplasia area.
Figure 15 shows evaluation by immunocytochemistry of purified anti-GRPR antibody of the present invention. The arrow indicates the presence of naked nuclei, suggestive of high-grade lesion, strongly marked.

### Detailed Description of the Invention

The present invention has as a common inventive concept to its various objects: using anti-GRPR antibodies to prepare kits or processes that comprise the binding, detection and / or quantification of GRPR receptors in biological tissues or cells, for the diagnosis of dysplastic and / or neoplastic lesions, including intraepithelial neoplastic lesions of the cervix and cervical cancer lesions.

For better understanding the invention, definitions are provided below for some of the terms and / or phrases used in this patent application.
GRPR

The term is used in this patent application as an abbreviation for Gastrin-Releasing Peptide Receptor.

### Anti-GRPR

### antibodies

The expression, in the context of the present application, refers to any anti-GRPR antibody, comprising both polyclonal antibodies and monoclonal antibodies. The anti-GRPR antibodies obtained with the use of non-natural peptide of the invention are a particularly useful embodiment for making kits for the detection and / or quantification of GRPR receptors in biological tissues or cells, for the diagnosis of dysplastic lesions and / or various other neoplastic lesions not limited to uterine.

### Control antibody

The control antibody refers to a polyclonal anti-GRPR antibody restricted for use in research, exclusively for immunohistochemistry, being its application not compatible with immunoprecipitation techniques such as, for example, Western blot. That is, the control antibody refers to an antibody available in the prior art and presents several problems as mentioned above, which are solved by the anti-GRPR antibody proposed by the present invention.

### Screening

The term "screening", in this application, should be understood differently from the term "diagnosis". It should be understood that it is a screening mode of identification of possible carriers of a given disease and, therefore, a primary identification of possible carriers of a particular disease (for example, in a population), this condition will be confirmed by diagnostic stage by a health professional. However, it should be clear that, in the context of the present patent application, screening is to help diagnose the condition and is not therefore diagnostic of said pathology.

### Material or biologic fluid, biological

### sample

The expression, in the context of the present patent application, comprises biological fluids such as, but not limited to, blood, saliva, sweat, cerebrospinal fluid. Also comprises cytological samples or biopsies of neoplastic or pre-neoplastic lesions, for example, cervical intraepithelial neoplasia and cervical cancer. Still in the context of the present patent application, the biological material may comprise elements such as cDNA, RNAs and / or proteins, whole or partial found in cells and / or tissues and / or organs of a eucaryote organism and / or a prokaryotic or obtained synthetically and identical to those found in the body.

### Dysplastic lesions

The expression, in the context of the present application, refers to abnormalities in cellular and tissue growth, leading to an uncontrolled growth of cells, tissues and / or organs of mammals, including humans. The dysplastic lesions comprise cervical intraepithelial neoplasia (CIN I, II and III, including ASC-H and ASCUS).

### Neoplastic lesions

The expression, in the context of the present application, refers to any abnormality in cell growth and / or cell division and comprises both benign neoplasia and malignant neoplasia. In the context of the present application, it should be understood that the neoplastic lesions can also be used as a synonym of tumors, both benign and malignant. Neoplastic lesions can be caused or be associated with various cancers.

### Immunocromatographic

### techniques

The expression, in the context of the present application, refers to any technique of separation and identification of soluble antigens. Among the methods comprise: thin layer chromatography with diffusion of antigens via an antibody solution.

### Means of contacting and / or detecting antigens

The expression, in the context of the present application, refers to any type of medium (material) needed to contact and / or detect the presence of antigens in the sample, for example, a membrane for lateral flow immunochromatography test and reagents for preparation of this material and sample, among others.

### Animal

The expression, in the context of the present application, refers to living beings eukaryotes, heterotrophs, and multicellular, with biological tissues such as nerve and muscle tissue, among others. A nonlimiting example of animal includes mammals and birds.

### Mammal

The expression, in the context of the present application, refers to vertebrates of which the females have mammary glands, such as, for example, rabbits, mice, horses, cattle and sheep, among others.

### Bird

The expression, in the context of the present application, refers to vertebrates and oviparous animals, such as chicken.

It is an object of the present invention an anti-GRPR antibody obtained from a process comprising the steps of:
a) exposing at least one animal to a peptide with at least 60% identity to the sequence SEQ ID NO: 1; and
b) obtaining anti-GRPR antibodies after the animal immune response to said peptide of step a).

In one embodiment, the peptide has at least 70% identity to the sequence SEQ ID NO: 1.

In one embodiment, the peptide has at least 80% identity to the sequence SEQ ID NO: 1.

In one embodiment, the peptide has at least 90% identity to the sequence SEQ ID NO: 1.

In one embodiment, the peptide is the sequence of SEQ ID NO: 1.

It is another object of the present invention a process for obtaining anti-GRPR antibody comprising the steps of:
a) exposing at least one animal to a peptide with at least 60% identity to the sequence SEQ ID NO: 1; and
b) obtaining anti-GRPR antibodies after the animal immune response to said peptide of step a).

In one embodiment, the peptide has at least 70% identity to the sequence SEQ ID NO: 1.

In one embodiment, the peptide has at least 80% identity to the sequence SEQ ID NO: 1.

In one embodiment, the peptide is encoded by the sequence SEQ ID NO: 2.

In one embodiment, the peptide is the sequence of SEQ ID NO: 1.

It is another object of the present invention a method / process of in vitro detection of dysplastic and / or neoplastic cervical lesions and comprising the steps of:
a) contacting at least one anti-GRPR antibody as disclosed by the present invention with at least one sample of biological material of uterine origin; and
b) detecting the binding of said antibody to one or more receptors present GRPR (s) in the said biological material.

In one embodiment, detecting the binding of step b) is by immunochromatography, immunohistochemistry or immunocytochemistry.

In one embodiment, in step b) there is at least one control marker for qualitative and / or quantitative comparison.

In one embodiment, in step b) there is at least one control marker for quantitative comparison.

In one embodiment, the control marker is an anti-GRPR polyclonal antibody distinct from the obtained in the present invention.

It is another object of the present invention the use of anti-GRPR antibody as disclosed by this invention for the preparation of kits for the detection and / or quantification of GRPR receptors in biological tissues or cells, being the kit for screening and / or diagnosis of dysplastic and / or neoplastic lesions.

It is another object of the present invention a kit for the qualitative and / or quantitative detection of dysplastic and / or neoplastic cervical lesions, comprising the anti-GRPR antibody as revealed by the present invention and at least one additional reagent for detecting the binding of said receptor antibody (s) with GRPR (s) present in tissues, cells, or biological fluids.

In one embodiment, the detection of the binding of the anti-GRPR antibody revealed by the present invention is / occurs by immunohistochemistry, immunocytochemistry or immunochromatography.

In one embodiment, the detection of anti-GRPR antibody disclosed by the present invention is / occurs by immunochromatography comprising capture assays.

It is another object of the present invention, a genetic construct for expression or production of antigenic peptide, said gene construct comprising:
a) a nucleotide sequence encoding a polypeptide having identity equal to or higher than 60% to SEQ ID NO: 1;
b) a promoter for the said nucleotide sequence; and
c) a nucleotide sequence selected from: a nucleotide transcription terminator sequence; a marker selection; a sequence secretion signal; a sequence that facilitates the export or purification; a nucleotide sequence encoding another polypeptide antigen sequence; combinations thereof or a plasmid comprehending sequences, being heterologous any of the nucleotide sequences defined above.

In one embodiment, the nucleotide sequence encoding a polypeptide having identity equal to 70% to SEQ ID NO: 1.

In one embodiment, the nucleotide sequence encodes a polypeptide with an identity of 80% to SEQ ID NO: 1.

In one embodiment, the nucleotide sequence encodes a polypeptide having identity equal to 90% to SEQ ID NO: 1.

In one embodiment, the nucleotide sequence encodes a polypeptide sequence SEQ ID NO: 1.

In one embodiment, the nucleotide sequence encoding the antigenic peptide comprises 45 to 60 nucleotides.

In one embodiment, the nucleotide sequence encoding the antigenic peptide is a nucleotide sequence having homology and / or identity greater than 60% compared to SEQ ID NO: 2.

In one embodiment, the nucleotide sequence encoding the antigenic peptide is the nucleotide sequence represented by SEQ ID NO: 2.

It is another object of the present invention a process for obtaining antibodies and comprising the steps of:
a) inoculating the gene construction as disclosed in this patent application in at least one animal; and
b) obtaining antibodies in said animal plasma after the immune response of the animal.

The examples shown below are intended only to illustrate some of the many ways to implement the invention without, however, limiting the scope of the same.

### Exemple 1. Development and obtaining of antigenic peptide

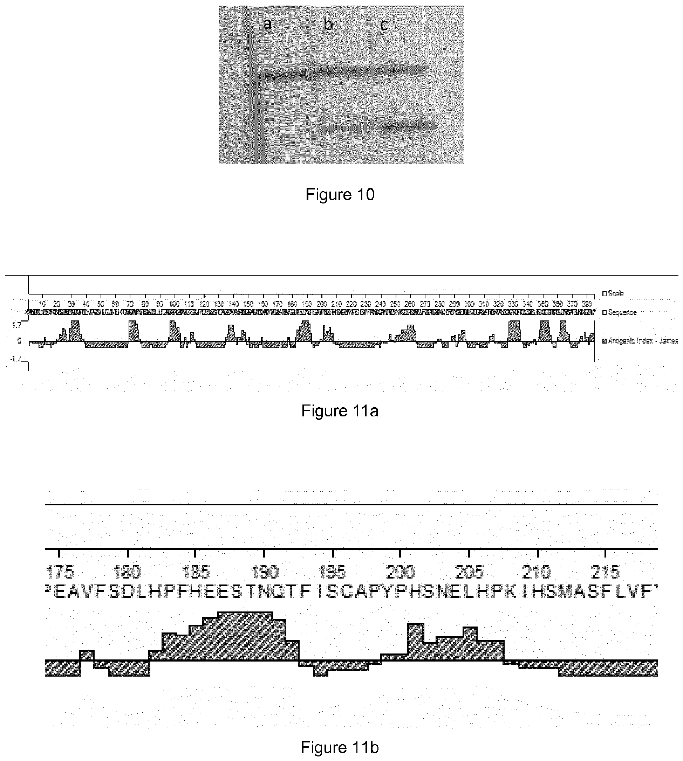

To identify the most immunogenic sequence of the GRP receptor it was used an antigenic index. It is an algorithm used to create a contour profile of the linear surface of a protein, which via a computer program generates values for surface accessibility parameters and combines these values with those obtained for the flexibility of the polymer chain and regional predicted secondary structure. This is because most, if not all the antigenic sites are located within the surface exposed regions of a protein. Thus, it was possible to identify the region included in the second extracellular loop following ESTNQTFISCAPYPHSN (Glu-Ser-Thr-Asn-Gln-Thr-Phe-Ile-Ser-Cys-Ala-Pro-Tyr-Pro-His-Ser-Asn) as the most immunogenic, as can be seen in figures 11 a and 11 b. The main difference of the natural peptide GRPR is the number of amino acids which has been reduced to the optimal size (and does not exist free in nature) for generating immunization (13 to 18 amino acids).

The sequence of peptide ESTNQTFISCAPYPHSN (Glu-Ser-Thr-Asn-Gln-Thr-Phe-Ile-Ser-Cys-Ala-Pro-Tyr-Pro-His-Ser-Asn), also in this application understood as the sequence SEQ ID NO: 1 was synthesized on solid phase using the Fmoc method (9-fluorenylmethoxycarbonyl) as described by Hirata et al (1994) and characterized by high-pressure analytical liquid chromatography (HPLC) (Shimadzu, Kyoto, Japan) in a reverse phase C18 Econosil column (5µm, 4.6 x 150mm) coupled to pump LC-8 type a, equipped with UV-Vis detector at 220nm in two solvent systems: 1 (A) TFA / H2O (1: 1000) and (B) TFA / ACN / H2O (1: 900: 100) 2 (A) H3PO4 / H2O (1: 1000) and (B) H3PO4 / H2O / ACN (1: 100: 900)

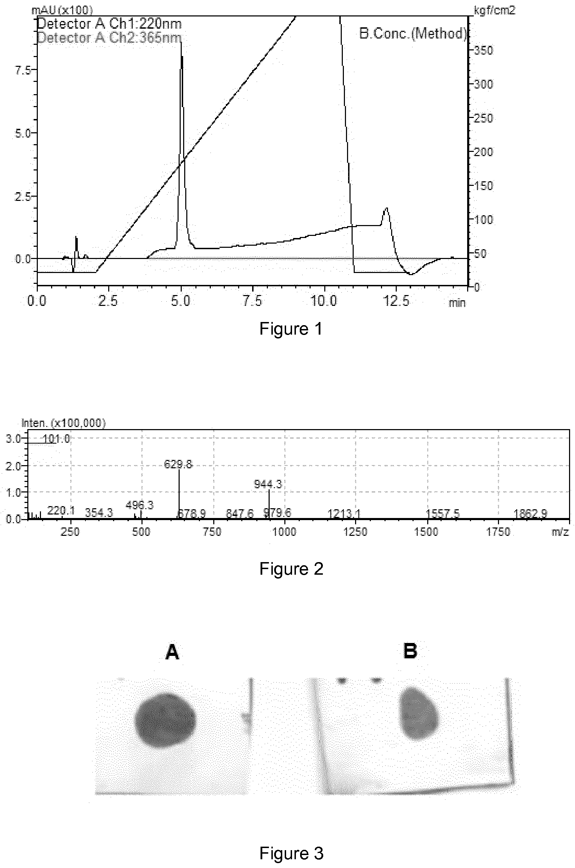

Next, the peptide was purified by semi-preparative high pressure liquid chromatography (Shimadzu) on a reverse phase C18 Econosil column (5µm, 22.5 x 250 mm) coupled pumps Model LC-8A equipped with detector UV-Vis at 220 nm in two solvent systems: (A) TFA / H2O (1: 1000) and (B) TFA / ACN / H20 (1: 900: 100)
The results can be seen in Figure 1.

The synthesized peptide was analyzed by mass spectrometry with the measuring method of the ion flight time mass spectrometry on a model LT Microflex (Bruker Daltonics) having MALDI-TOF technology (Matrix Laser Desorption Ionization Time-of-Flight) ( Figure 2). In all analyzes, the matrix used was the α-cyano-4-hidroxicianamínico acid, amino acid analysis and if necessary, Edman degradation.

### Example 2. Development and obtaining of antibody

After synthesis of the peptide generated from the ESTNQTFISCAPYPHSN sequence, it was carried the development of a polyclonal antibody by rabbit immunization.

On the first day the rabbit received subcutaneous inoculation of 200 ug of antigen emulsified in an equal volume of oily adjuvant. In ten day intervals two additional inoculations of the antigen emulsified in adjuvant were given. Five days after the last dose, the serum was collected and tested by dot blot for the detection of specific antibodies against the corresponding antigen.

### Dot-Blot

For immunodetection and quantitation of the antibody through dot blot, the protein (approximately 0.5 ug) was applied to nitrocellulose membrane of 0.45 micrometers. After drying for 30 minutes at room temperature, the membrane was blocked with blocking solution for 1 hour and incubated for 2 hours with serial dilutions of serum diluted in 5% blot. Next, the membrane was washed three times for 10 minutes with 5% blot and incubated with mouse-phosphatase conjugate anti-IgG diluted 1/5000 in 5% blot for 90 minutes at room temperature, then washed three times with PBS and stained for 10 minutes. The result can be seen in Figure 3.

### Example 3. Development of kit and method for the qualitative and / or quantitative detection of dysplastic and / or neoplastic cervical lesions- Immunohistochemistry

After obtaining the rabbit polyclonal antibody, laboratory tests were performed using immunohistochemical technique. Cervical tissue paraffin blocks with neoplastic and non-neoplastic tissue of the cervix They were selected and immunohistochemical technique was proceeded. All samples were tested with the antibody of the present invention and a control polyclonal anti-rabbit GRPR antibody restricted for use in research (Affinity BioReagents, Golden, CO, USA catalog number OPA1-15619).

Briefly, after dewaxing, inactivation of endogenous peroxidase and cross-reacting with normal blocking serum, 4 micron sections were incubated overnight at 4 ° C with diluted primary antibody (1: 200 antibody of the invention and 1:50 control antibody). The identification of the location of the primary antibody was achieved by subsequent application of biotinylated antibody, conjugated streptavidin peroxidase (LSAB, Dako, and tetrahydrochloride / H2O2 diamino benzidine; DAB Kit, Dako). A cancer sample in known positive pancreas to GRPR was used as a positive control and the negative control was obtained by omission of the primary antibody. In immunohistochemical analysis, the lesions were considered positive if there was moderate or strong staining of GRPR in more than 10% of the cells. Conversely, the lesions were considered negative if there was weak staining in less than 10% of the cells. Comparisons were made between the two antibodies, relating to staining intensity, presence of nuclear or cytoplasmic staining marking and distinct cell populations. All samples were analyzed independently by two pathologists "blind" to the previous diagnosis. In case of disagreement, a consensus was reached with the participation of a third pathologist.

### Results in immuno-histochemistry

Initially we used the concentration of the antibody of the invention similar to that used with the control antibody, or 1:50. However, at this concentration, the antibody of the invention showed a much stronger color than the control antibody. Therefore, for an equivalent comparison of the antibody of the invention with the control antibody, we used a dilution of 1: 200 in antibody of the invention and control antibody at 1:50, reaching the same intensity of color. This result demonstrates the superiority of the antibody of the invention, which has the ability to mark cells expressing GRPR in more dilute concentrations compared to the control antibody. This has great impact in terms of cost-effectiveness, and allows the performance of 4 times more tests with the antibody of the invention than the control antibody. The sensitivity is closely related to the inherent characteristic method with the minimum amount of antigen that can be detected. Thus, it is suggested that the antibody of the invention has higher sensitivity in detecting GRPR compared to the control antibody.

It is noteworthy that the polyclonal antibodies obtained also differ from control anti-GRPR antibodies given the fact that the anti-GRPR control antibodies are exclusively used for research and for immunohistochemical technique, not being possible the use of immunoprecipitation techniques such as, for example, Western blot.

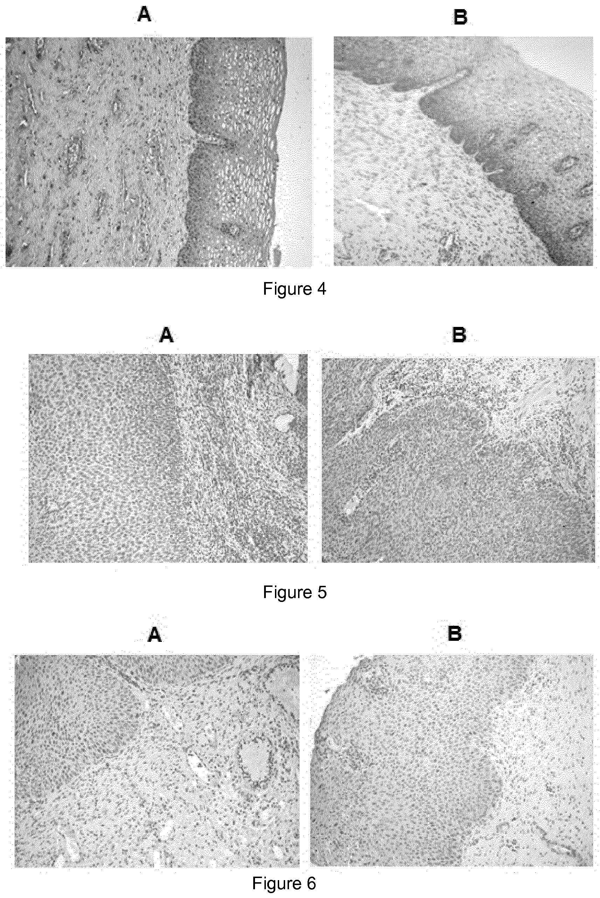

Comparison of primary antibodies (invention and control) in relation to GRPR expression by immunohistochemistry in non-neoplastic cervical samples, samples with cervical intraepithelial lesion II (CIN II), cervical intraepithelial lesion III (CIN III) and cervical intraepithelial II lesion (CIN II) can be observed in figures 4 to 7, respectively. It can also be seen that the antibody of the invention exhibits both cytoplasmic and nuclear staining, whereas the control antibody (prior art) basically stains the cytoplasm of cells. This gives a great advantage to the antibody of the invention, allowing a superiority in the diagnosis of dysplastic and neoplastic lesions.

Another marked difference between the antibody obtained in the present invention and control antibody is the antibody titer. The antibody of the invention can be diluted 4 times compared to control antibody, and yet confer adequate intensity for detection of altered cells or tissues.

Therefore, the results and experimental tests presented in the patent application clearly indicates the superiority of polyclonal antibodies obtained in relation to the existing anti-GRPR control antibody in the prior art and such superiority is also proven by the rapid immunoassay revealed by following example 4.

### Example 4. Kit and development process for the qualitative and / or quantitative detection of dysplastic and / or neoplastic cervical lesions - immunochromatographic rapid test

Samples of cervical smears of women with cervical lesions were obtained. These samples were collected with cytobrush and stored in 0.9% saline in 2 ml vials in a temperature of - 200C. At the same time cervical smears were collected for conventional cytological analysis and immunocytochemistry analysis with GRPR. For immunochromatographic tests only positive samples were used, according to cytological examination (CIN I, II and III) and the presence of GRPR (over 5 stained cells per slide). After defrosting the solution, lateral flow immunochromatography tests were performed.

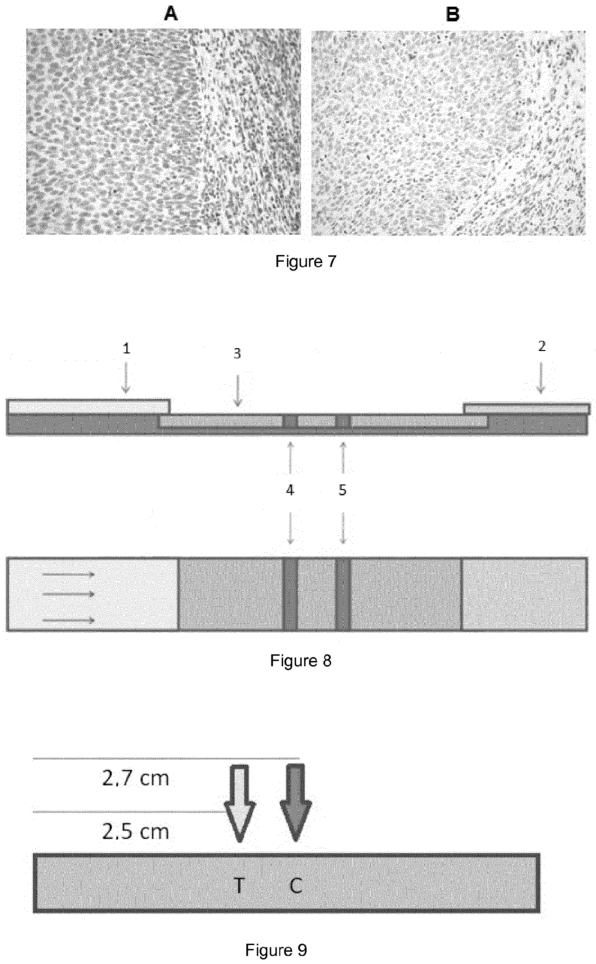

For immunochromatography testing we used the FUSION5 ™ membrane, Whatman, GE. In a general way, immunochromatographic tests for lateral flow are constructed in accordance with Figure 8. However, the FUSION5TM membrane dispenses insertion of the receiving and absorbing portions of the samples, being constituted of a single strand.

### Preparation of the conjugate for the

### test line:

For the conjugation of primary polyclonal anti-GRPR to colloidal gold (62R-GC010, Fitzgerald) we used the direct method. A single incubation with 10 nM colloidal gold and 10 g / ml of primary antibody was done for one hour, and placed in phosphate buffer 10 mM pH 7.2. This solution was applied to the capture zone 2.5 cm from the beginning of the strip, with the aid of a pipette.

### Preparation of the control line:

For the control line it was used a secondary goat anti-rabbit IgG antibody conjugated to colloidal gold 15nM (43R-IG085GD, Fitzgerald) in a solution containing TBS, pH 8.2, 1% BSA, 15 mM NaN 3 and 20% glycerol. This solution was applied on the membrane with a pipette 2.7 cm from the beginning of the strip. After applying these two lines, the material was allowed to dry for at least 3 hours at 37°C. Then the material was cut into 5 mm strips with the aid of a blade. A schematic drawing can be seen in Figure 9.

For performing the test, samples were defrosted at room temperature. The solution containing the sample was transferred to a conical tube and the brush was washed again in 2 ml of saline. The final volume was centrifuged at 2,700 g for 10 minutes. After, the supernatant was removed and added to 100 mL of running buffer (Tris, NaCl, KCI, EDTA, Triton X-100, Tween 20 and sodium azide BSA). 100 uL of this solution was applied in the initial portion of the strip previously prepared. As a control, in some strips the running buffer was applied without adding the sample of cervical smears. After 10 minutes, visual reading was carried out.

### Results:

It was noticed that the samples flowed until the end of the strips. The figure 10 shows that the test and control lines exhibited staining in the first minute after application of the solution, but achieved greater intensity after 5 minutes of the beginning of the test. In the control strip, only the control line showed positive staining, indicating the release of the secondary antibody.

It is clear in this application that the developed kit is an important tool for screening and diagnosis of cervical lesions, including intraepithelial neoplastic lesions of the cervix and cervical cancer.

### Example 5. Gene construct for expression of an antigenic peptide

The gene construct of the invention is useful for the expression or production of antigenic peptide. In one embodiment, the construct comprises the nucleotide sequence SEQ ID NO: 2. Figure 11 illustrates this construction. The nucleotide sequence SEQ ID NO: 3, in turn, is only the complete sequence comprising the nucleotide sequence SEQ ID NO: 2.

### Example 6. Anti-GRPR antibody evaluation by immunoassay using the Western blot technique

The immunoassay technique of Western blotting was performed as previously described in the literature. Cell lysis was performed in two known GRPR positive samples: a cell line derived from colorectal cancer commercially available, HT-29, and a cervical lesion sample. Briefly, the samples were pipetted in a concentration of 1 ug / ml in polyacrylamide gel and electrophoresis was proceeded to transfer to PVDF membrane, followed by blocking the membrane with milk powder MTTBS. The activation with anti-GRPR antibody occurred overnight. After, membrane were washed in TTBS, and secondary anti-rabbit IgG antibody was added for 1h 20min. The revelation was made with ImmobilonTM Western Chemiluminescent HRP Substrate (Millipore) in chemiluminescence detector ImageQuant LAS500.

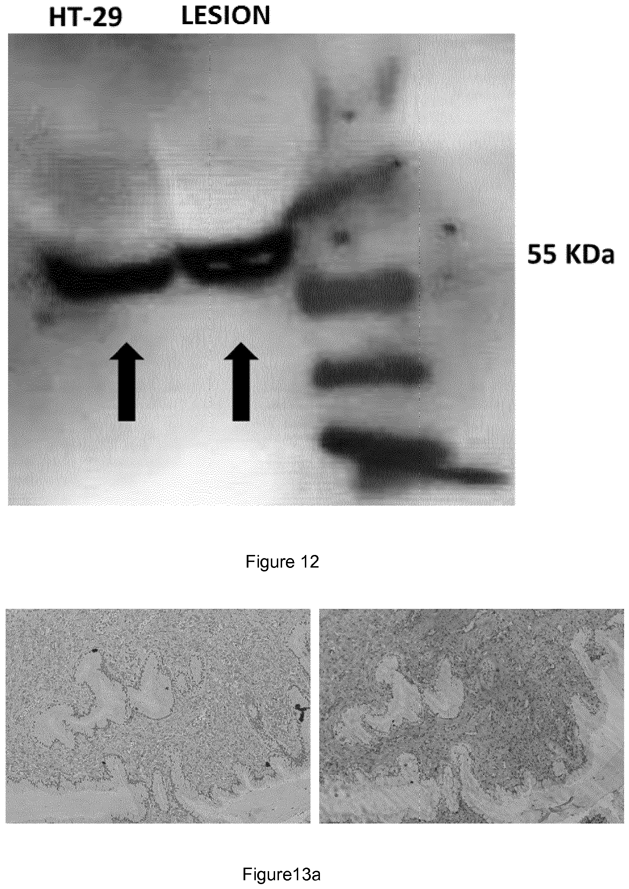

As can be seen in Figure 12, the anti- GRPR antibody showed positivity in both samples, demonstrating its activity through Western blot. The results can be visualized by the presence of bands of approximately 55 kDa. The Western blot technique was also carried out with the control antibody, without displaying any positive result. This results of lack of positivity with control antibody was expected, since the antibodies for research use are not suitable for this type of immunoprecipitation technique. Therefore, the antibody of the present invention has a clear advantage compared to the control antibody (prior art), since it enables the realization of other techniques, for example, Western blot.

The anti-GRPR antibody of the present invention was purified by affinity chromatography purification with protein G. The adsorption buffer used was sodium phosphate buffer 20 mM pH 7 and eluted antibody, 0.1M glycine buffer at pH 2.7. After, the sample was dialyzed in phosphate buffered saline (PBS) pH 7.2.

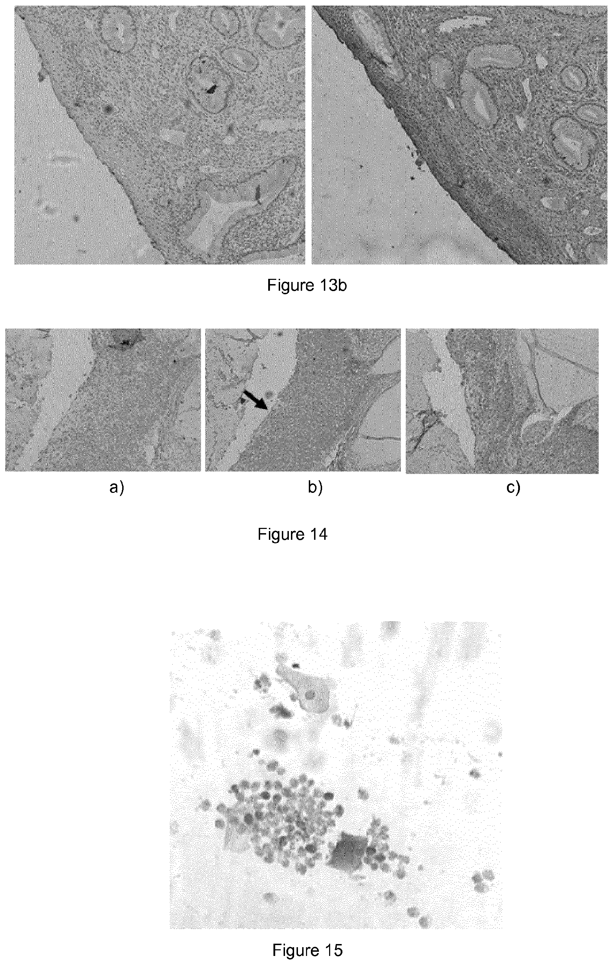

After purification of the antibody of the invention, there were new comparative analysis with the control antibody. We evaluated 25 cervical intraepithelial lesions of high and low grade, as well as invasive cancer. All samples were positive for expression of the antibody of the invention, which can be seen in comparison with hematoxylin-eosin (HE) staining (Figures 13a and 13b). This was not observed with the control antibody, where some lesions showed no staining.

It was observed that the antibody disclosed by the present invention also exhibited cytoplasmic and nuclear staining, whereas the control antibody exhibited predominantly cytoplasmic staining, of weaker intensity.

In Figure 14 one can observe staining with HE (a), intense cytoplasmatic and nuclear staining with the antibody of the present invention (b), and weak staining, predominantly cytoplasmic, with the control antibody (c). Another important observation was that the antibody disclosed by the present invention showed intense nuclear staining as aggravation of lesions, ie, GRPR expression was more intense in high-grade CIN 3 lesions, which was not observed with the antibody control.

Furthermore, immunocytochemical analyzes were performed on Pap smears of patients with cervical intraepithelial lesions. There was intense cytoplasmic and nuclear staining in the smear cells that showed changes suggestive of high-grade lesions (Figure 15).

Below is a table that summarizes the comparison of the antibody of the present invention and the control antibody:

**Tabel 1. Comparison of the usual anti-GRPR antibodies of the prior art and the antibodies obtained in the present invention.**

| **Antibody use** | **Anti-GRPR antibodies commonly used in research** | **Anti-GRPR antibodies obtained in the present invention** |
|---|---|---|
| Research | Yes | Yes |
| Diagnosis | No | Yes |
| Immunocytochemistry | No | Yes |
| Immunohistochemistry | Yes | Yes |
| Western Blot | No | Yes |
| Rapid test | No | Yes |

Those skilled in the art will value the knowledge presented herein, and may reproduce the invention presented in the embodiments and other embodiments which fall within the scope of the appended claims.

## Claims

1. Anti-GRPR antibody **characterized by** being obtained from a process comprising the steps of:
a) exposing at least one animal to a peptide with at least 60% identity to the sequence SEQ ID NO: 1; and
b) obtaining anti-GRPR antibodies after the animal immune response to said peptide of step a).

2. Antibody according to claim 1, **characterized by** the peptide having at least 90% identity to the sequence SEQ ID NO: 1.

3. Antibody according to claim 1 **characterized by** the peptide being the sequence of SEQ ID NO: 1.

4. Process for obtaining anti-GRPR antibody **characterized by** comprising the steps of:
a) exposing at least one animal to a peptide with at least 60% identity to the sequence SEQ ID NO: 1; and
b) obtaining anti-GRPR antibodies after the animal immune response to said peptide of step a).

5. Process of in vitro detection of dysplastic and / or neoplastic cervical lesions **characterized by** comprising the steps of:
a) contacting at least one anti-GRPR antibody as defined in any one of claims 1 to 3 with at least one sample of biological material of uterine origin; and
b) detecting binding of said antibody to one or more GRPR receptors present in the said biological material.

6. Process according to claim 5, **characterized by** detecting the binding of step b) by immunochromatography, immunohistochemistry or immunocytochemistry.

7. Process according to claim 5 or 6, **characterized in that** step b) comprising at least one control marker for qualitative and / or quantitative comparison.

8. Process according to claim 7 **characterized by** the control marker being a distinct anti-GRPR antibody from the antibody as defined in any one of claims 1 to 3.

9. Anti-GRPR antibody use as defined in any one of claims 1 to 3 **characterized by** being for the preparation of kit for the detection and / or quantification of GRPR receptors in biological tissues or cells, and being the kit for screening and / or diagnosis of dysplastic and / or neoplastic lesions.

10. Kit for the qualitative and / or quantitative detection of dysplastic and / or neoplastic uterine lesions **characterized by** comprising the anti- GRPR antibody as defined in any one of claims 1 to 3 and at least one additional reagent for detecting binding of said antibody to GRPR receptor (s) present in tissues, cells, or biological fluids.

11. Kit according to claim 10, **characterized by** detecting binding of the antibody as defined in any one of claims 1 to 3 by immunohistochemistry, immunocytochemistry or immunochromatography.

12. Kit according to claim 11, **characterized by** immunochromatography detection comprising capture assays.

13. Construction gene for expression or production of antigenic peptide **characterized by** said gene construct comprising:
a) a nucleotide sequence encoding a polypeptide having identity equal to or higher than 60% to SEQ ID NO: 1;
b) a promoter for the said nucleotide sequence; and
c) a nucleotide sequence selected from: a nucleotide transcription terminator sequence; a selection marker; a sequence secretion signal; a sequence that facilitates the export or purification; a nucleotide sequence encoding another antigenic polypeptide sequence; combinations thereof or a plasmid comprising such sequences, being heterologous any of the nucleotide sequences defined above.


LIST OF SEQUENCES
<110> Ziel Biosciences Ltda.
<120> ANTI-GRPR ANTIBODY, PROCESS OF OBTAINING, PROCESS OF
DETECTION, ANTIBODY USE, KIT AND GENIC CONSRUCT
<130> GRPR PCT
<160> 3
<170> PatentIn version 3.5
<210> 1
<211> 17
<212> PRT
<213> Artificial Sequence
<220>
<223> GRPR plus (derived from GRPR gene), function: antigenic peptide
<400> 1
Glu Ser Thr Asn Gln Thr Phe Ile Ser Cys Ala Pro Tyr Pro His Ser
1               5                   10                  15
Asn
<210> 2

<211> 51
<212> DNA
<213> Artificial sequence
<220>
<223> antigenic peptide coding
<400> 2
gaaagcacca accagacctt cattagctgt gccccatacc cacactctaa t 51
<210> 3
<211> 2681
<212> DNA
<213> Artificial Sequence
<220>
<223> antigenic peptide coding
<400> 3
aaaggtatct gttaagctag gtaggaactg cagtcggctg gttgcttctc atctggagaa 60
agcaggcaac tgggcagtga ttgaagtgtc cagcaggggg ctggcattct ctgtctataa 12
gtaacactgg ttcctcttca gagcctcagc tcagcggagc tgccgtttgc tggtgaagcc 180
cgtgacgtgc aaagcatcct gcctatagga tttgaggatt tctcagtgca gtttttttct 240
acccacttta aacctccaga ttctaaatat caggaaagac gctgtgggaa aatagcaggc 300
caaaagttct tagtaaactg cagccaggga gactcagact agaatggagg tagaaagaac 360
tgatgcagag tgggtttaat tctaagcctt tttgtggcta agttttgttg ttgttaactt 420
attgaattta gagttgtatt gcactggtca tgtgaaagcc agagcagcac cagtgtcaaa 480
atagtgacag agagttttga ataccatagt tagtatatat gtactcagag tatttttatt 540
aaagaaggca aagagcccgg catagatctt atcttcatct tcactcggtt gcaaaatcaa 600
tagttaagaa atagcatcta agggaacttt taggtgggaa aaaaaatcta gagatggctc 660

taaatgactg tttccttctg aacttggagg tggaccattt catgcactgc aacatctcca 720
gtcacagtgc ggatctcccc gtgaacgatg actggtccca cccggggatc ctctatgtca 780
tccctgcagt ttatggggtt atcattctga taggcctcat tggcaacatc actttgatca 840
agatcttctg tacagtcaag tccatgcgaa acgttccaaa cctgttcatt tccagtctgg 900
ctttgggaga cctgctcctc ctaataacgt gtgctccagt ggatgccagc aggtacctgg 960
ctgacagatg gctatttggc aggattggct gcaaactgat cccctttata cagcttacct 1020
ctgttggggt gtctgtcttc acactcacgg cgctctcggc agacagatac aaagccattg 1080
tccggccaat ggatatccag gcctctcatg ccctgatgaa gatctgcctc aaagccgcct 1140
ttatctggat catctccatg ctgctggcca ttccagaggc cgtgttttct gacctccatc 1200
ccttccatga ggaaagcacc aaccagacct tcattagctg tgccccatac ccacactcta 1260
atgagcttca ccccaaaatc cattctatgg cttcctttct ggtcttctac gtcattccac 1320
tgtcgatcat ctctgtttac tactacttca ttgctaaaaa tctgatccag agtgcttaca 1380
atcttcccgt ggaagggaat atacatgtca agaagcagat tgaatcccgg aagcgacttg 1440
ccaagacagt gctggtgttt gtgggcctgt tcgccttctg ctggctcccc aatcatgtca 1500
tctacctgta ccgctcctac cactactctg aggtggacac ctccatgctc cactttgtca 1560
ccagcatctg tgcccgcctc ctggccttca ccaactcctg cgtgaacccc tttgccctct 1620
acctgctgag caagagtttc aggaaacagt tcaacactca gctgctctgt tgccagcctg 1680

gcctgatcat ccggtctcac agcactggaa ggagtacaac ctgcatgacc tccctcaaga 1740
gtaccaaccc ctccgtggcc acctttagcc tcatcaatgg aaacatctgt cacgagcggt 1800
atgtctagat tgacccttga ttttgccccc tgagggacgg ttttgcttta tggctagaca 1860
ggaacccttg catccattgt tgtgtctgtg ccctccaaag agccttcaga atgctcctga 1920

gtggtgtagg tgggggtggg gaggcccaaa tgatggatca ccattatatt ttgaaagaag 1980
ccatcaagtc ttaagttttt catttcaact tgtgaacgtt tcttctgatg tgaagcaaac 2040
cttccctttt cagaaaaggg aacaagtaga aaattatttt ttaagcctca agccctgtta 2100
aatggtcgtg gccaattatg tcatagaaac tgtatgaaca accagattta catagcagag 2160
aaatcataca ttgaatgctt actttgtgaa agacttcacc ttgtcatttc tttaagcaga 2220
cgctagtact ttagaaatat aacttgactc tgttttcagg aatatctgta atacacaaac 2280
caaggaacaa cttttattta cactcctaat atgaaaagtc aatcctgtga gagagctcca 2340
tgtatgaggg acactctcca agttgataac aatggaagcg agtttaatat aaaacaattc 2400
cctaagcatt tatttttttt ttaaaaagat gttactgagg acctagaaga aatgctcaat 2460
acatactttg aaagcaaaaa tacaatcaaa cacattgaca cgtatataaa gatccacgcg 2520
tggctgtgcg tgatatctca cactctgaat tcttacttga tggaggtttt gtttgctgct 2580
acggttttaa tcatccaggg tgccattcca ccatagaaga gcaatccttt taggaaaaaa 2640
aaaatcatgc tattaattaa tcaaatatct ataaatgcat a 2681